# EUROPÄISCHE PATENTSCHRIFT

(11) **EP 1 863 928 B1**
(45) Veröffentlichungstag und Bekanntmachung des Hinweises auf die Patenterteilung: **16.02.2011**
(21) Anmeldenummer: 06725094.4
(22) Anmeldetag: 16.03.2006
(51) Int. Cl.: C12Q 1/68

(54) **VERWENDUNG VON GENAKTIVITÄTS-KLASSIFIKATOREN FÜR DIE IN VITRO KLASSIFIZIERUNG VON GENEXPRESSIONSPROFILEN VON PATIENTEN MIT INFEKTIÖSEM/NICHTINFEKTIÖSEM MULTIORGANVERSAGEN**
USE OF GENE ACTIVITY CLASSIFIERS FOR THE IN VITRO CLASSIFICATION OF GENE EXPRESSION PROFILES OF PATIENTS WITH INFECTIOUS/NON-INFECTIOUS MULTIPLE ORGAN FAILURE
UTILISATION DE CLASSIFICATEURS D'ACTIVITE GENETIQUE POUR LA CLASSIFICATION IN VITRO DE PROFILS D'EXPRESSION GENETIQUE DE PATIENTS PRESENTANT UNE DEFAILLANCE MULTIORGANIQUE INFECTIEUSE OU NON-INFECTIEUSE

(30) Priorität: 21.03.2005 DE 102005013013
(43) Veröffentlichungstag der Anmeldung: 12.12.2007
(73) Patentinhaber: SIRS-Lab GmbH, 07745 Jena (DE)
(72) Erfinder: RUSSWURM, Stefan, 07743 Jena (DE); REINHART, Konrad, 07743 Jena (DE)
(74) Vertreter: Winter, Brandl, Fürniss, Hübner, Röss, Kaiser, Polte - Partnerschaft
(86) Internationale Anmeldenummer: PCT/EP2006/060780
(87) Internationale Veröffentlichungsnummer: WO 2006/100203

(56) Entgegenhaltungen:
- US-A1- 2004 096 917
- DATABASE EMBL Homo sapiens cDNA clone 13. Januar 1996 (1996-01-13), XP002412098 gefunden im EBI Database accession no. N32857
- DATABASE EMBL Homo sapiens cDNA clone 13. Januar 1996 (1996-01-13), XP002412099 gefunden im EBI Database accession no. N32853
- DATABASE EMBL Homo sapiens cDNA clone 13. Januar 1996 (1996-01-13), XP002412100 gefunden im EBI Database accession no. N32495
- DATABASE EMBL Homo sapiens cDNA clone 4. Juni 1999 (1999-06-04), XP002412101 gefunden im EBI Database accession no. AI701077
- DATABASE EMBL Human anti-hepatitis A Ig lambda chain 10. März 1992 (1992-03-10), XP002412102 gefunden im EBI Database accession no. M87790
- DATABASE EMBL Homo sapiens cDNA clone 25. März 1999 (1999-03-25), XP002412103 gefunden im EBI Database accession no. AI559317
- DATABASE EMBL Homo sapiens cDNA clone 19. Januar 1996 (1996-01-19), XP002412104 gefunden im EBI Database accession no. N34897
- DATABASE EMBL Homo sapiens cDNA clone 14. April 1998 (1998-04-14), XP002412105 gefunden im EBI Database accession no. AA907084
- DATABASE EMBL Homo sapiens cDNA clone 17. Februar 1996 (1996-02-17), XP002412106 gefunden im EBI Database accession no. N45223
- "AMERICA COLLEGE OF CHEST PHYSICIANS/SOCIETY OF CRITICAL CARE MEDICINE CONSENSUS CONFERENCE: DEFINITIONS FOR SEPSIS AND ORGAN FAILURE AND GUIDELINES FOR THE USE OF INNOVATIVE THERAPIES IN SEPSIS" CRITICAL CARE MEDICINE, WILLIAMS AND WILKINGS COMPANY, BALTIMORE, MA, US, Bd. 20, Nr. 6, Juni 1992 (1992-06), Seiten 864-874, XP008036965 ISSN: 0090-3493
- COBB J.P. ET AL.: "SEPSIS GENE EXPRESSION PROFILING: MURINE SPLENIC COMPARED WITH HEPATIC RESPONSES DETERMINED BY USING COMPLEMENTARY DNA MICROARRAYS" CRITICAL CARE MEDICINE, WILLIAMS AND WILKINGS COMPANY, BALTIMORE, MA, US, Bd. 30, Nr. 12, Dezember 2002 (2002-12), Seiten 2711-2721, XP008037048 ISSN: 0090-3493
- TSUKAHARA Y. ET AL.: "GENE EXPRESSION IN HUMAN NEUTROPHILS DURING ACTIVATION AND PRIMING BY BACTERIAL LIPOPOLYSACCHARIDE" JOURNAL OF CELLULAR BIOCHEMISTRY, WILEY-LISS INC, US, Bd. 89, Nr. 4, 1. Juli 2003 (2003-07-01), Seiten 848-861, XP009063472 ISSN: 0730-2312

### Sequenzprotokoll

<110> SIRS-Lab GmbH
<120> Verfahren zur Unterscheidung zwischen nichtinfektiösen und infektiösen Ursachen eines Multiorganversagens
<130> 85/SL0528/PC
<140> Current Application Number
   <141> 16. März 2006
<160> 1297
<170> Patent Prepare 0.3.5
<210> 1
   <211> 670
   <212> DNA
   <213> Homo sapiens
<220>
   <221> misc_feature
   <222> (503)..(503)
   <223> n is a, c, g or t.
<220>
   <221> misc_feature
   <222> (530)..(530)
   <223> n is a, c, g or t.
<220>
   <221> misc_feature
   <222> (564)..(564)
   <223> n is a, c, g or t.
<220>
   <221> misc_feature
   <222> (669)..(669)
   <223> n is a, c, g or t.
<400> 1
   gttattggac gaggtgtatt aaaataaaat caaaaataga aatattaggt atatacacaa 60
   cagcataagt gaaacacatc atgtgataca gaagtttcca ttcaaagctt acaggagaaa 120
   aatttagtga acagaaccgc ttgaacctcc tgccaccgtg gcttattaga gccttgtagt 180
   cacttatgtt ccttcatatc cttgggtata aatagtctca tacacatgct acacaattta 240
   gaaaagattg ctgaatcata aaaatagccc cgaatgtggc actttagctt tctagagtac 300
   aactaaaatt aatacttgat ctttaacata atgttcaaat atttgaagca acaaaggaaa 360
   ctcttatgtt ttagaaggcc ctataaggtt cttcagagat tagaatccta gttcttccaa 420
   actacattgt caaaacaaat ctggatatag gaaattgtgg aaatggaaaa gctggactcc 480
   taaaaatatt ccctctaaca ccngagcagt ttctggaggc tcttctcccn ttcctgggag 540
   gtccaggtga gcccggtgcc ttcngggtga gcagacaggg cagctgtggt gctcacgggc 600
   tcccctgctg gaagagagag agtgtgccgc cactcctatg ggatgtctag caggttgagc 660
   agggtcaana 670
<210> 2
   <211> 609
   <212> DNA
   <213> Homo sapiens
<220>
   <221> misc_feature
   <222> (490)..(490)
   <223> n is a, c, g or t.
<220>
   <221> misc_feature
   <222> (495)..(495)
   <223> n is a, c, g or t.
<220>
   <221> misc_feature
   <222> (516)..(516)
   <223> n is a, c, g or t.
<220>
   <221> misc_feature
   <222> (552)..(552)
   <223> n is a, c, g or t.
<220>
   <221> misc_feature
   <222> (564)..(564)
   <223> n is a, c, g or t.
<220>
   <221> misc_feature
   <222> (589)..(589)
   <223> n is a, c, g or t.
<400> 2
   ggcaaggaat aacaaaataa tagaaactcc atatatatta gaaaaagatc tgtaagtttg     60
   agccttatat attattacaa tgtggcaatc aagaggaaaa ctcaagacaa ctgtgcgctg    120
   tgcttgtcct gttttgttca aaccacgata attttaaata aatagtaatg ttcaacattc    180
   actgatgaca ttatctatca accacatcac acctggaaca cagtagataa taaatggcag    240
   ctgttatacc ttccttcttg aaacaaaatc ctgtcatttc attctcattt taactttgtg    300
   ttaagcttct acaatccata aaaatgataa aacaataaaa tggtccaaca ttatctagtc    360
   cagtcttatt tttacaaaac attgacttta aaagtccctg gagattttaa ttttactggt    420
   ttaatctgtc ttccctaccc ttgtttaaaa aaaaatagaa tcatgtgtca ttattgggtt    480
   gagtacccgn tatcnggaat ccttgggaca gtggtnttgg attccaatat ggaatattgg    540
   cttatattac cngtgagtat cccnatctgg acacgggaat cctaatgcnc gtggacatcc    600
   cttgagtgt                                                            609
<210> 3
   <211> 599
   <212> DNA
   <213> Homo sapiens
<220>
   <221> misc_feature
   <222> (495) .. (495)
   <223> n is a, c, g or t.
<220>
   <221> misc_feature
   <222> (527)..(527)
   <223> n is a, c, g or t.
<220>
   <221> mise_feature
   <222> (539)..(539)
   <223> n is a, c, g or t.
<220>
   <221> mise_feature
   <222> (598)..(598)
   <223> n is a, c, g or t.
<400> 3
   tgcttattga tttttttaag tctgagggaa attacccaaa atgtgtcatt ccttaacatt      60
   taaaaatatg ttacataaaa atgagcaact gtgataacaa ataaaacttg gagagaaaaa    120
   agttggctat ttccttattc aaggagacag agctaccaga atacatcaag atagcgcaca    180
   aattttttaa atccactgcc acttctgctg aaacaaaagt caactgataa gaaaattccc    240
   attcaaaaca attttaattg tactgttaac tcttcctcca ttttttaatt tcatgaagtc    300
   ataatccaat ctgagttggt ctgttgggca ttagcagaca tagtcactgg agaggagtga    360
   gatgatgtca gagcatgggg cacagtattt ataaatgtct cctttaagaa agtgtaagta    420
   gtatcaagtt cagctttcaa tgggtaagaa atccccaata ccagttgtgt cagcaggaag    480
   gaggagctct tgaanggatg gagagatttg gatgggcccg tgatccnatt ggattggtna    540
   acctgcattt tccccgttaa caagccggtg tggaagtccg gtttccaagg ctgccatnt     599
<210> 4
   <211> 436
   <212> DNA
   <213> Homo sapiens
<400> 4
   ttttgagata gagtcttact gtgtcaccaa ggctgcaggg caatggcctg atcttgactc      60
   actgcaacct cagcctcccg agtagctggg actacaggca tgcgccacca cacccagcta     120
   cctttttttg atgttgttgt atttctagta gagacagggt ttcgccatat tggccaggat     180
   ggactctaac tcctgacctc cagtgatcca cctgcctcgg cctcccaaag tgttgggatt     240
   acaggcatga gccaccacgc cccacctgac tcccagtctc ttattaccca acctgcaatt     300
   ttcaaaaccc cagtccgact gctaaccttt gacctcttgg atttccctcc ccgcactccc     360
   ctatctggat gaaggaccca cctcaggttc ttgttgaagc tcagatcttg taccgctgtc     420
   ttcttgatct ctaccc                                                     436
<210> 5
   <211> 877
   <212> DNA
   <213> Homo sapiens
<400> 5
   caagaggcag cgctctcggg acgtctccac catggcctgg gctctgctgc tcctcaccct     60
   cctcactcag gacacagggt cctgggccca gtctgccctg actcagcctg cctccgtgtc    120
   tgggtctcct ggacagtcga tcaccatctc ctgcactgga accaacaatg atgttgggag    180
   ttataacctt gtctcctggt accagcagca cccaggcaaa gcccccaaaa tcatgattta    240
   tgaggtcagt aagcggccct caggggtttc taatcgcttc tctggctcca agtctggcaa    300
   cacggcctcc ctgacaatct ctgggctcca ggctgaggac gaggctgatt attactgctg    360
   ctcatatgca ggtagttaca ctgtggtttt cggcggaggg accaaactga ccgtcctagg     420
   tcagcccaag gctgccccct cggtcactct gttcccgccc tcctctgagg agcttcaagc    480
   caacaaggcc acactggtgt gtctcataag tgacttctac ccgggagccg tgacagtggc     540
   ctggaaggca gatagcagcc ccgtcaaggc gggagtggag accaccacac cctccaaaca     600
   aagcaacaac aagtacgcgg ccagcagcta tctgagcctg acgcctgagc agtggaagtc     660
   ccacagaagc tacagctgcc aggtcacgca tgaagggagc accgtggaga agacagtggc     720
   ccctacagaa tgttcatagg ttctaaaccc tcaccccccc cacgggagac tagagctgca     780
   ggatcccagg ggaggggtct ctcctcccac cccaaggcat caagcccttc tccctgcact     840
   caataaaccc tcaataaata ttctcattgt caatcac                              877
<210> 6
   <211> 371
   <212> DNA
   <213> Homo sapiens
<400> 6
   tttttttttt tttttttttt tttttttttt tttttttttt tttttttttt tttttttttt     60
   tcacccccca aaaaaaatta aaaattaaaa aaaaaaagaa acacaccggc ctcccttttg    120
   gggaaaaaaa ggggccccca aaacccccaa agggggggga cctttgggaa aggcccccaa     180
   agggtttgga aaaaagtttt gggtttgggc ccaatttggg tttaaaaaaa aaaccccctt 240
   tcaaaaacca ataaaatttt aacccaggaa aacggggggg ggggcccccg ggaaaaaacc 300
   cttttttggg gggccctttt tttttttttt acccaggggc ccccccaaaa aggggggggg 360
   gggggggggg g 371
<210> 7
   <211> 301
   <212> DNA
   <213> Homo sapiens
<220>
   <221> misc_feature
   <222> (165)..(165)
   <223> n is a, c, g or t.
<400> 7
   taacatgtgg tgaagtttaa ttattcaata atggttccaa ttattaaaca tgactattgc 60
   ataatgacat aattggctgt ggatgtgctt ttgtgtggcc acataaactc caggaaactt 120
   tgtcttcatt agtttcatta gtcttcatga ggtcaggcct ggggnagctc gtttgactct 180
   tcccaagtgt ggggtaggtg ttgatgcctt gagagtgcca gagtgacaca ggtcatgacc 240
   cacgccaggg tgaagtgaga actctcaggg gtcacctctc cttgttccac atctgtcttt 300
   t 301
<210> 8
   <211> 587
   <212> DNA
   <213> Homo sapiens
<400> 8
   ttatgcttgc tttttattta gagatttttc ataggctgca gtttcacact gaataaattt 60
   gtttctgtat ctacaccatg aacatacacg atgacagcaa gtatttattg aacaccacct 120
   cataatatgt gctcgaccca gctctcagtg ctctctgcaa gcctcagtcc ttgaagtcac 180
   ccctgagagg cagaagctgt cactgttccc ctggcactgg ggagaaaagg gacatgtagg 240
   ggctgttctg ttgcaagttt agagatccac gtggtcagcg cctcaacgct ggttccatca 300
   gacagagata ccggacccgt ctaggattcc gttgttggcc gcaagtgcag aggcgttcgc 360
   catacttgtt ggagatgtcc aaattgcagt ttgcttcaca gagggccacc acgataggca 420
   tgttgcaatc tttacattgc ccatggaggg gagtattgcc gtgcctgtct tgataatcga 480
   cgaaacaccc tggttgagga gagtcttgat tacctccatc tgacaccgtc taaagccaga 540
   tgaagggcaa tgtgtccgtc cttgtcgcaa gcattaaggt cggctcc 587
<210> 9
   <211> 532
   <212> DNA
   <213> Homo sapiens
<220>
   <221> misc_feature
   <222> (477)..(477)
   <223> n is a, c, g or t.
<400> 9
   caacttctat ggtggtttta ttatgaaaca aaataaacct ttcaatatca tattcaaatt     60
   atcaaaaata tagctcaaac cttcctacac tgataaaaat aatatatatc attcacatca    120
   acatcatgac agctcaaagt tagctatttg aaataaatca tcatactttt gaaagctact    180
   cttattacat ttctaaacag agtgctattt ttcctttttc taagagaaac tataaagcat    240
   accccaaagt actcacattc tggctacatt tggaaaacta tttgaaagat taaaccttca    300
   tgtttatctc atctcacaat ttacatttat gtattaattt aaatggctac agaactattt    360
   aaaaatttat tttataaaca tggtactaca aattttaaaa cttcctgttc taaaagtaac    420
   tatttaaaaa gatttctgga acacaccatc aaaaagatac tgttagaaaa tttcccnaga    480
   gtggacacac accaggcaaa tccaggccgg aggaattaag acgggtttag ca            532

## Beschreibung

Die vorliegende Erfindung betrifft die Verwendung von Genaktivitätsmarkern zur Klassifizierung von Patienten die an einem infektiösem bzw. nicht infektiösem Multiorganversagen erkrankt sind.

Die Erfindung betrifft weiterhin die Verwendung dieser Genaktivitäts-Klassifikatoren als gespeicherte Werteparameter in Vorrichtungen, welche zur *in vitro* Diagnose für Patienten mit infektiösem bzw. nicht infektiösem Multiorganversagen verwendet werden. Außerdem betrifft die Erfindung eine Vorrichtung zur *in vitro* Diagnose von Patienten, die an infektiösem bzw. nicht infektiösem Multiorganversagen erkrankt sind.

Weiterhin betrifft die Erfindung die Verwendung der Genaktiviätsmarker/- Klassifikatoren für die Klassifizierung von Genexpressionsprofilen von Patienten zur Beurteilung der Therapieeffekte von Wirkstoffen gegen infektiöses bzw. nicht infektiöses Multiorganversagen.

Trotz Fortschritten im pathophysiologischen Verständnis und der supportiven Behandlung stellt das Multiorgandysfunktionssyndrom (MODS) bzw. das Multiorganversagen (MOV) bei intensivmedizinischen Patienten die häufigste Todesursache dar und nimmt weltweit weiter zu. Die Folgen dieser Entwicklung sind nicht nur für den einzelnen Patienten erheblich, sondern haben enorme Auswirkungen auf die Kosten des Gesundheitswesens und den medizinischen Fortschritt in vielen Bereichen der Medizin. Als Multiorganversagen bezeichnet man das gleichzeitig oder in rascher zeitlicher Abfolge auftretende Versagen von zwei oder mehr vitalen Organsystemen. Das Multiorgandysfunktionssyndrom (MODS) geht als initiale Organinsuffizienz dem MOV voraus [1]. Man spricht heute vom Multiorganversagen wenn zwei oder mehr Organe gleichzeitig oder nacheinander Funktionstörungen aufweisen, wobei ein chronisch persistierendes Organversagen auszuschließen ist [2]. Die Prognose des MOV hängt eng mit der Anzahl der beteiligten Organsysteme zusammen. Die Mortalität beträgt bei Versagen eines Organs innerhalb der ersten 24 Stunden 22%, nach 7 Tagen 41%. Beim Versagen von drei Organsystemen steigt die Mortalität am ersten Tag auf 80% und nach 4 Tagen auf 100% an [3].

Zur klinischen Schweregradeinteilung des MODS und MOV wird regelmäßig der Multiple-Organ-Failure-Score (MOF-Score) von GORIS et al. [4] oder alternativ auch der Sepsis-related Organ Failure Assessment-(SOFA-) Score verwendet [5]. Der MOF-Score erlaubt eine schnelle und klinisch einfache Klassifikation der Organfunktion in drei Abstufungen. Ein MOF-Score > 4 wird in der klinischen Literatur regelmäßig als MOV bezeichnet [6]. Der SOFA-Score ist ein Punktesystem, welches die schnelle klinische Beurteilung der Funktion, jeweils in vier Schweregradstufen, folgende Organsysteme bewertet: Atmung (Lunge), Koagulation, Leber, Herz-Kreislaufsystem, zentrales Nervensystem und Niere.

Das MOV läuft klinisch in drei Phasen ab [7]:
1. Organ im Schock: Der auslösende pathophysiologische Mechanismus ist ein Perfusionsdefizit unterschiedlichster Genese. Dieses Geschehen spielt sich innerhalb von Stunden ab und hinterläßt noch keine bleibenden Schäden.
2. Organdysfunktion: Ein fortbestehendes Perfusionsdefizit innerhalb der nächsten Tage führt zur Entstehung eines SIRS (Systemic Inflammatory Response Syndrome, klassifiziert nach [8]) mit lokalen Ödemen und Zellschädigungen. Diese Phase wird als Multiorgandysfunktionssyndrom (MODS) bezeichnet.
3. Organversagen: Das fortbestehende Perfusionsdefizit führt zur Stase im Splanchnikusgebiet, wodurch es zur Superinfektion und Translokation von Endotoxinen aus dem Darm kommt. Das führt zu einer Potenzierung der klinischen Symptome und zum Vollbild der Sepsis. Aus der Organdysfunktion wird ein Organversagen.

MODS und MOV sind Krankheitsbilder mit einer komplizierten Pathophysiologie. Bis heute sind die genauen molekularen Ursachen der Entstehung und die Komplexizität der immunologisch-inflammatorischen Wirtsantwort auf schwere Infektion und Trauma, die zur Auslösung eines SIRS und zu den entsprechenden kardiozirkulatorischen Auswirkungen führen kann, nur unzureichend verstanden [9].

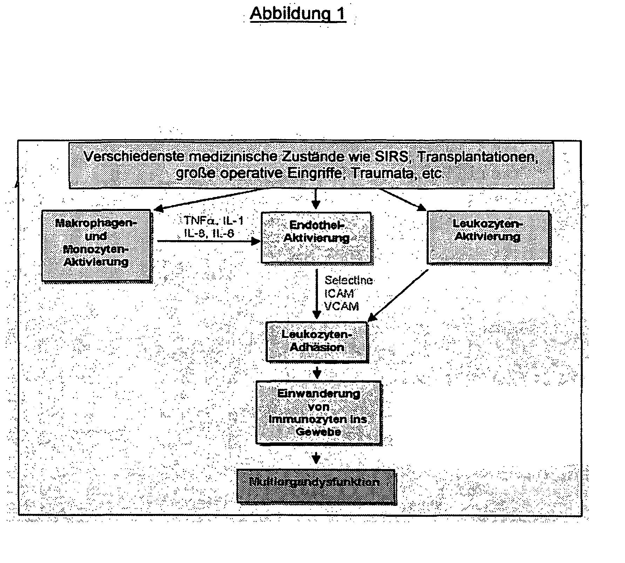

MODS und MOV können kann sowohl infektiologischer als auch nichtinfektiologischer Genese sein. MODS und MOV treten regelmässig als klinisch wichtige Komplikation bei Patienten mit Sepsis, nach traumatischen Schock, bei Patienten nach Operationen unter Einsatz der Herz-Lungen-Maschine, nach Organtransplantationen u.v.m. auf (Abbildung 1). Ein wichtiger Pathomechanismus für die Entstehung von MODS und MOV ist die Entwicklung eines systemischen Inflammationssyndromes (SIRS, [8]). Die im Rahmen eines SIRS initiierten pathophysiologischen Prozesse involvieren nicht nur alle Komponenten des Immunsystems, sondern beeinträchtigen das kardiozirkulatorische System in allen Ebenen und beschränken sich nicht nur auf Myokarddepression und Vasodilatation. Die kardiozirkulatorischen Veränderungen vor allem auf Ebene der Mikrozirkulation bilden die gemeinsame Endstrecke und resultieren in einer Gewebehypoxie, die als wichtiger Kofaktor in der Pathogenese des Multiorganversagens gilt.

Abbildung 1 beschreibt exemplarisch die aus heutiger Sicht wichtigsten Mechanismen der Entstehung von MODS und MOV [10]: Ein überaktives Immunsystem scheint bei der Entstehung des Multiorganversagens eine zentrale Rolle zu spielen. Dabei nimmt das Endothel durch Sekretion von Zytokinen und durch Vermittlung der Leukozyten-Adhäsion eine zentrale Schlüsselrolle ein. In den Endothelzellen werden Signaltransduktionskaskaden aktiviert, die letztendlich zur Expression und Aktivierung von Transkriptionsfaktoren führen.

Das noch lückenhafte Wissen über die Abläufe in der Frühphase des MODS und MOV ist der Grund dafür, das es bis heute keine sensitive/spezifische Diagnostik, die zwischen infektiösen und nichtinfektiösen Ursachen diskriminieren kann, existiert. Neuartige Biomarker und Diagnostika, nunmehr auch auf Genexpressionsebene, können die für die Früherkennung des Multiorganversagens sowie zur Unterscheidung zwischen infektiösen und nichtinfektiösen Ursachen eines MODS und MOV essentiellen diagnostischen Informationen liefern und stellen darüber hinaus einen wichtigen Beitrag zur Aufklärung der pathophysiologischen Mechanismen systemischer Inflammationen dar.

Die klinisch häufig benutzten Frühsymptome wie Fieber, Leukozytose, Tachykardie und Tachypnoe sind bei der Diagnose eines MODS oder MOV sowie bei der Unterscheidung zwischen infektiösen und nichtinfektiösen Ursachen eines MODS und MOV völlig unspezifisch. Parameter, welche die Mikrozirkulationsstörungen früh erfassen, wie pH-Veränderungen der Drammukosa [11] und Laktatspiegel im Kapillarbett [12,13], das Auftreten einer respiratorischen Insuffizienz, deren Ursache nicht in der Lunge liegt [2],der Anstieg der Leukozyten-Elastase [14,15], die Höhe des Neopterin-Spiegels [16], die Aktivierung polymorphkemiger Leukozyten und die Höhe des IL-6-Spiegels [17] sind als Frühparameter für die spätere Entstehung von MODS und MOV bedingt geeignet, können aber keinen Beitrag zur Unterscheidung zwischen infektiösen und nichtinfektiösen Ursachen eines MODS und MOV machen. Es besteht daher ein dringender Bedarf für neue diagnostische Verfahren, welche die Fähigkeit des Fachmanns verbessern sollen, nichtinfektiöses von infektiösen MODS oder MOV frühzeitig zu unterscheiden, und dem Ansprechen auf spezifische Behandlungen Aussagen abzuleiten.

Genau die Unterscheidung zwischen infektiösen und nichtinfektiösen Ursachen eines MODS und MOV ist aber von höchster medizinischer Bedeutung, da mittels einer solchen Unterscheidung z.B. Antibiotika spezifischer eingesetzt werden können, was neben einer Vermeidung von Nebenwirkungen durch den unspezifischen Einsatz von Antibiotika auch zu einer erheblichen Kosteneinsparung beiträgt. Gleichfalls können so den Patienten sehr belastende sowie Zeit- und Personal-intensive diagnostische Massnahmen (z.B. Transport zum CT/MRT) zur Identifizierung des jeweiligen Infektionsortes, die Durchführung umfangreicher mikrobiologischer Methoden (z.B. die Untersuchung von Blutkulturen, die ebenfalls mit der Abnahme großer Mengen an Blut verbunden sind) aber auch der risikoreiche Austausch aller mit dem Patienten verbundener Plastikmaterialien wie Venenkatheder etc. bei Vorliegen eines nichtinfektiösen MODS oder MOV vermieden werden. Vice versa kann die schnelle Identifikation infektiöser Ursachen eines MODS oder MOV die zeitnahe Einleitung solcher Maßnahmen und damit die Reduktion der Sterblichkeit sicherstellen.

Technologische Fortschritte, insbesondere die Entwicklung der Microarray-Technologie, versetzen den Fachmann nun in die Lage, 10000 oder mehr Gene und deren Genprodukte gleichzeitig zu vergleichen. Die Anwendung solcher Microarray-Technologien kann Hinweise auf den Status von Gesundheit, Regulationsmechanismen, biochemischer Wechselwirkungen und Signalübertragungsnetzwerken geben. Das Verbessern des Verständnisses darüber, wie ein Organismus auf Infektionen reagiert, sollte die Entwicklung von verstärkten Erkennungs-, Diagnose- und Behandlungsmodalitäten für Infektions-Erkrankungen erleichtern.

Microarrays stammen vom "Southern blotting" [19] ab, was die erste Herangehensweise darstellt, DNA-Moleküle in einer räumlich ansprechbaren Art und Weise auf einer festen Matrix zu immobilisieren. Die ersten Microarrays bestanden aus DNA-Fragmenten, oft mit unbekannter Sequenz, und wurden auf eine poröse Membran (normalerweise Nylon) punktweise aufgebracht. Routinegemäß wurden cDNA, genomische DNA oder Plasmid-Bibliotheken verwendet, und das hybridisierte Material wurde mit einer radioaktiven Gruppe markiert [20-22].

Heute erlaubt die Verwendung von Glas als Substrat und Fluoreszenz zur Detektion zusammen mit der Entwicklung neuer Technologien für die Synthese und für das Aufbringen der Nukleinsäuren in sehr hohen Dichten, die Nukleinsäurearrays zu miniaturisierten bei gleichzeitiger Erhöhung des experimentellen Durchsatzes und des Informationsgehaltes [23-25].

Eine Begründung für die Anwendbarkeit der Microarray-Technologie wurde zunächst durch klinische Untersuchungen auf dem Gebiet der Krebsforschung geliefert. Hier haben Expressionsprofile ihre Nützlichkeit bei der Identifizierung von Aktivitäten einzelner Gene oder Gengruppen gezeigt, die mit bestimmten klinischen Phänotypen korrelieren [26]. Durch die Analyse vieler Proben, die von Individuen mit oder ohne akute Leukämie oder diffusen B-Zell Lymphomen stammten, wurden Genexpressionsmarker (RNA) gefunden und anschließend für die klinisch relevante Klassifizierung dieser Krebsarten angewandt [26,27]. Golub et al. haben herausgefunden, daß verlässliche Vorhersagen nicht aufgrund von irgendeinem einzelnen Gen gemacht werden können, aber daß Vorhersagen, die auf der Veränderung der Transkritiption von 53 Genen (ausgewählt aus über 6000 Genen, die auf den Arrays vertreten waren) basieren, sehr genau sind [26].

Aus WO 03/002763 ist bekannt, dass die Messung der Genexpression mittels Microarrays grundsätzlich für die Diagnose von Sepsis und sepsisähnlichen Zuständen verwendet werden können.

Die grundsätzliche Verwendbarkeit von Genexpressionsprofilen, welche beispielsweise mittels der Microarray-Technik erhalten werden können, zur Diagnose von SIRS, generalisierten inflammatorischen Entzündungen, Sepsis und schwerer Sepsis ist in den deutschen Patentanmeldungen der Anmelderin der vorliegenden Erfindung DE 103 40 395.7, DE 103 36 511.7, DE 103 150 31.5 sowie DE 10 2004 009 952.9 beschrieben.

Von Feezor et al. [28] ist bekannt, dass sich die Genaktivitäten von Patienten, welche aufgrund ihrer operativen Behandlung ein SIRS mit Multiorgan Dysfunction Syndrome (MODS) entwickelten, gegenüber Patienten, die unter den gleichen operativen Bedingungen eine SIRS ohne MODS entwickelten, unterscheiden. Diese Untersuchungen lassen jedoch keine Aussage über die Unterscheidung von nichtinfektiösen MOV gegenüber infektiösen MOV zu, da in bei den Patienten keine Infektion nachgewiesen wurde.

Für die Klassifizierung von Genexpressionsprofilen sind bereits mehrere Methoden und deren Anwendung für Genexpressionsdaten, wie beispielsweise lineare und quadratische Diskriminierungsanalysen, Compound Covariant Predictor, Nearest Neighbor Classification, Classification trees oder Support Vector Machines, beschrieben worden [26, 29, 30, 31, 32]. Eine allgemeinen Überblick über die Anwendung von Klassifizierungsmethoden für die Analyse Genexpressionsdaten ist in [33] dargestellt.

Ziel der Klassifikationsmethoden ist die Entwicklung von multivarianten Klassifikatoren, die eine Vorhersage über die Zugehörigkeit eines neuen Datensets zu einer Klasse ermöglichen. Damit können beispielsweise Patienten anhand von Klassifikatoren hinsichtlich ihrer Reaktion auf eine spezielle Behandlung zu Respondem bzw. Non-Respondern klassifiziert werden.

Die Entwicklung von Klassifikatoren verläuft prinzipiell in drei Schritten: 1. Die Auswahl von statistisch relevanten Features aus einen großen Datensatz. Für Genexpressionsanalysen werden zunächst anhand von univarianten Tests zwischen mehreren Klassen die statistisch relevanten Gene, basierend auf ihrem Expressionsmuster als Features ausgewählt. 2. Die Bestimmung der Klassifikatoren mit Hilfe verschiedener Methoden zur Klassifizierung an deren Ende ein Trainingsset an Klassifikatoren zur Verfügung steht. 3. Die Validierung dieses Trainingssets anhand von neuen, nicht klassifizierten Testsets von Genexpressionsprofilen und die Optimierung des Trainingssets.

In WO 2004/108957 wird allgemein die Klassifizierung von Biomarkem (Nukleinsäuren) und deren Verwendung für die Diagnose von SIRS bzw. Sepsis beschrieben. Eine Klassifizierung und/oder Verwendung der Biomarker für die Diagnose von infektiösen zw. nicht infektiösen Multiorganversagen wird nicht beschrieben.

In der nicht vorveröffentlichten deutschen Patentanmeldung DE 102004 049897 werden erstmals Genaktiviätsmarker zur Unterscheidung zwischen infektiösem und nicht infektiösem Multiorganversagen gezeigt. Darin wird die Verwendung von 1297 verschiedene Genen für die in vitro Diagnose von Patienten, die an einem infektiösem bzw. nicht infektiösem Multiorganversagen erkrankt sind, beschrieben.

Die vorliegende Erfindung geht über den in DE 102004049897.041 gezeigten Stand der Technik hinaus, indem anhand von spezifischen Untersuchungen der Genexpressionsprofile von Patientproben ermittelt wurde, welche Genaktiviäten sich als Klassifikatoren für die *in vitro* Diagnose zur Unterschiedung von infektiösem und nicht infektiösem Multiorganversagen eignen.

Ausgangspunkt für die in der vorliegenden Patentanmeldung offenbarten Erfindung ist die Erkenntnis, daß sich anhand von Genaktivitäts-Klassifikatoren, die Genexpressionsprofile von Patienten mit nichtinfektiösem MOV bzw. mit infektiösem MOV gruppieren lassen. Die Verwendung dieser Klassifikatoren ist mit den bisher zur Diagnose verwendeten klinischen Parametern nicht möglich, aber für die Einleitung einer spezialisierten intensivmedizinischen Therapie sehr bedeutungsvoll.

Der vorliegenden Erfindung liegt somit die Aufgabe zugrunde, durch die Verwendung von Genaktivitäts-Klassifikatoren zwischen nicht infizierten Patienten ohne Multiorganversagen, einem nichtinfektiösen Multiorganversagen und einem infektiösen Multiorganversagen zu unterscheiden.

Die Aufgabe der Erfindung wird durch Genaktivitätsmarker gemäß Anspruch 1, ein Verfahren zur Klassifizierung von Patienten gemäß Anspruch 6, ein Microarray gemäß Anspruch 9 und eine Vorrichtung gemäß Anspruch 10 gelöst.

Im folgenden wird für die Patienten, die intensivmedizinisch behandelt wurden, bei denen jedoch keine Infektion nachgewiesen werden konnte und kein Multiorganversagen diagnostiziert wurde der Begriff "ITS-Kontrolle" verwendet.

Insbesondere betrifft die vorliegende Erfindung die Verwendung von von Genaktiviäts-Klassifikatoren anhand derer die in vitro aus einer Patientenprobe erhaltenen Genexpressionsprofile nach nichtinfektiösen und infektiösen Multiorganversagens klassifiziert werden.

Ferner dient die vorliegende Erfindung unter Verwendung der Klassifikatoren der therapiebegleitenden Verlaufsbeurteilung von Patienten, welche an nichtinfektiösen und infektiösen Ursachen eines Multiorganversagens erkrankt sind.

Die erfindsgemäßen Genaktivitäts-Klassifikatoren sind ferner nützlich als Ein- oder Ausschlußkriterium von Patienten, die an nichtinfektiösen oder infektiösen Ursachen eines Multiorganversagens erkrankt sind, in klinische Studien der Phasen 2-4.

Eine bevorzugte Ausführungsform der Erfindung betrifft die zur Verfügungstellung von Genaktivitäts-Klassifikatoren für die elektronische Weiterverarbeitung sowie zur Herstellung von Software für die Beschreibung der individuellen Prognose eines Patienten, für Diagnosezwecke und/oder Patientendatenmangement-systemen. Die Genaktivitäts-Klassifikatoren werden hierfür als Grundlage für die automatische Beurteilung von zu untersuchenden Genexpressionsprofilen in diagnostischen Vorrichtungen *in vitro* verwendet. Die Genexpressions-Klassifikatoren sind hierbei als Werteparamater in einer Software, auf einem integriertem Schaltkreis, einem EPROM oder anderem dem Fachmann bekannten technischen Möglichkeiten zur Speicherung von Werteparametern fixiert.

Eine weitere bevorzugte Ausführungsform der Erfindung besteht in einer Vorrichtung, welche die als Werteparameter gespeicherten Genaktiviäts-Klassifikatoren verwendet, um eine *in vitro* Diagnose von Patienten mit nichtinfketiösem und infektiösem Multiorganversagen zu ermöglichen. Eine solche Vorrichtung beinhaltet neben den als Werteparameter gespeicherten Genaktiviäts-Klassifikatoren eine Möglichkeit, nicht klassifizierte Genexpressionsprofile von Patientenproben mit den gespeicherten Genaktiviäts-Klassifikatoren zu vergleichen und das resultierende Ergebnis als technischen Anzeige auszugeben. Der Vergleich kann beispielsweise durch die elektronische Verarbeitung von den als elektronische Signale umgewandelten Werteparamertern der Genaktiviäts-Klassifikatoren mit den elektronischen Signalen, welche aus den zu untersuchenden Genexpressionsprofilen gewonnen wurden, realisiert werden. Das resultierende Ergebnis dieses Vergleichs besteht in der Klassifizierung des zu untersuchenden Genexpressionsprofils in eine der Klassen nichtinfektiöses Multiorganversagen bzw. infektiöses Multiorganversagen. Als technische Anzeige kommen analoge und/oder digitale Anzeigen, wie beispielsweise Score-Systeme (ähnlich bereits in der in vitro Diagnostik verwendeten APACHE oder SOFA Score) oder *in silico* Gele, akustische Signale oder andere dem Fachmann bekannte Verfahren zum Einsatz.

In einer bevorzugten Ausführungsform ermöglicht dieses Vorrichtung außerdem auch die Erstellung von Genexpressionsprofilen zum Vergleich mit dem gespeicherten Genaktiviäts-Klassifikatoren. Hierfür besteht diese Vorrichtung aus einem Modul für die Probenvorbereitung der *in vitro* gewonnen Patientenprobe, einem Modul für die Hybridisierung der Patientenprobe mit Genaktiviätssonden die von den Genaktivitäts-Klassifikatoren abgeleitet sind, einem Modul für die Auslesung der Hybridisierungssignale, einem weiteren Modul für die Bildanalyse der ausgelesenen Hybridisierungssignale, einem Modul, dass den automatischen Vergleich mit dem gespeicherten Genaktiviätsklassifikatoren ermöglicht sowie einem Modul, dass die Anzeige des resultierenden Vergleichs ermöglicht. Es ist dem Fachmann selbstverständlich, dass je nach Automatisierungsgrad der Vorrichtung nicht alle Module in der Vorrichtung vereint sein müssen. Beispiele für Vorrichtungen, die bereits heute eine auto-/semiautomatische Erstellung von Genexpressionsprofile ermöglichen sind der Light Cycler der Fa. Roche, der Smart Cycler der Fa. Cepheid oder das AP-System der Fa. Clondiag Chip Technologies.

Es ist dem Fachmann weiterhin klar, dass sich außer dem in dieser Patentanmeldung beschriebenen Verfahren zur Erstellung von Genexpressionsprofilen mittels Microarray-Technologie, auch alle anderen Verfahren für Analysen der differentiellen Genexpression verwendet werden können.

Die erfindungsgemäßen Genaktiviäts-Klassifikatoren können auch zur Herstellung von "in silico" Expertensystemen und/oder zur "in silico"-Modellierung von zelluläreren Signalübertragungswegen verwendet werden.

Als Genaktiviäts-Klassifikatoren gemäß der vorliegenden Erfindung werden Gene und/oder Genfragmente verwendet, welche ausgewählt werden aus der Gruppe bestehend aus SEQ ID 1.1 bis SEQ ID I.9 sowie Genfragmenten davon mit wenigstens 5-2000, bevorzugt 20-200, mehr bevorzugt 20-80 Nukleotiden.

Diese Sequenzen mit der SEQ ID I.1 bis zur SEQ ID I.9 sind durch den Umfang der vorliegenden Erfindung mit umfaßt und sind in dem angefügten, 9 Sequenzen umfassenden, Sequenzprotokoll, das somit Bestandteil der Beschreibung der vorliegenden Erfindung ist, im Einzelnen offenbart. Das Sequenzprotokoll ist somit ebenfalls Bestandteil der Offenbarung der Erfindung. Dieses Sequenzprotokoll beinhaltet zudem eine Zuordnung der einzelnen Sequenzen mit der SEQ ID I.1 bis zur SEQ ID I.9 zu deren GenBank Accession Nr. (Internet-Zugang über http:/www.ncbi.nlm.nih.gov/).

Hierzu können ebenfalls hybridisierungsfähige synthetische Analoga der aufgelisteten Sonden verwendet werden.

Die Verwendung von Insertions-, Deletions- oder Nukleotidaustausch-Mutanten der Sequenzen mit der SEQ ID I.1 bis zur SEQ ID 1.9 für die Zwecke der vorliegenden Erfindung ist ebenfalls denkbar, solange diese Mutationen das Hybridisierungsverhalten der Sequenzen für die Zwecke der vorliegenden Erfindung nicht wesentlich verändern. Wird in der vorliegenden Erfindung auf Genaktivitätsmarker Bezug genommen, so sind solche Mutationen mit umfaßt.

In einer weiteren Ausführungsform werden die Genaktivitäts-Klassifikatoren mit der SEQ ID I.1 bis zur SEQ ID I:9 für die Klassifizierung von Genexpressionsprofilen aus Patientenproben mit nichtinfektiösem bzw. infektiösen Multiorganversagen gemäß der Tabelle 1 zu logischen Auswahlregeln verknüpft.

**Tabelle 1: Auswahlregeln für die Klassifizierung IST-Kontrolle, nichtinfektiöses bzw. infektiöses**

| MOV | | | |
|---|---|---|---|
| Klasse | ITS-Kontrolle | nichtinfektiöses Multiorganversagen | infektiöses Multiorganversagen |
| | (Seq-ID I.1)↑ | (Seq-ID I.3)↑ | (Seq-ID I.5)↑ |
| | and | and | and |
| | (Seq-ID I.3)↓ | (Seq-ID I.6)↓ | (Seq-ID I.7)↓ |
| Klassifikatoren | Or | or | or |
| | (Seq-ID 1.2)↑ | (Seq-ID I.4)↑ | (Seq-ID I.8)↑ |
| | and | and | and |
| | (Seq-ID I.4) ↓ | (Seq-ID 1.5)↓ | (Seq-ID I.9)↑ |

| | | | |
|---|---|---|---|
| ↑ überexprimierte Genaktiviät, ↓ unterexprimierte Genaktiviät | | | |

Eine weitere Ausführungsform der Erfindung ist dadurch gekennzeichnet, daß die in Anspruch 1 aufgelisteten Gene oder Genfragmente und/oder von deren RNA abgeleiteten Sequenzen ersetzt werden durch synthetische Analoga, Aptamere sowie Peptidonukleinsäuren.

Eine weitere Ausführungsform der Erfindung ist dadurch gekennzeichnet, daß die Probe ausgewählt wird aus: Körperflüssigkeiten, insbesondere Blut, Liquor, Urin, Ascitesflüssigkeit, Seminalflüssigkeit, Speichel, Punktat; Zellinhalt oder eine Mischung davon.

Eine weitere Ausführungsform der Erfindung ist dadurch gekennzeichnet, daß Zellproben gegebenenfalls einer lytischen Behandlung unterzogen werden, um deren Zellinhalte freizusetzen.

Es ist dem Fachmann klar, daß die in den Ansprüchen dargelegten einzelnen Merkmale der Erfindung ohne Einschränkung beliebig miteinander kombinierbar sind.

Als Genaktivitäts-Klassifikatoren im Sinne der Erfindung werden alle abgeleiteten DNA-Sequenzen, Partialsequenzen und synthetischen Analoga (beispielsweise Peptido-Nukleinsäuren, PNA) verstanden. Die auf Bestimmung der Genexpression auf RNA-Ebene bezogene Beschreibung der Erfindung stellt keine Einschränkung sondern nur eine beispielhafte Anwendung dar.

Die auf Blut bezogene Beschreibung der Erfindung stellt nur eine beispielhafte Anwendung der Erfindung dar. Als biologische Flüssigkeiten im Sinne der Erfindung werden alle Körperflüssigkeiten des Menschen verstanden.

Weitere Vorteile und Merkmale der vorliegenden Erfindung ergeben sich aufgrund der Beschreibung eines Ausführungsbeispiels, sowie anhand der Zeichnung.

Abbildung 1 zeigt den pathologischen Verlauf von Multiorganversagen, ausgehend von unterschiedlichen medizinischen Zuständen.

### Ausführungsbeispiel

Untersuchung zur Erstellung und Validierung von Genaktivitäts-Klassifikatoren zur Klassifizierung von Genexpressionsprofilen von Patientenproben in eine der Klassen: ITS-Kontrolle, nichtinfektiöses Multiorganversagen oder infektiöses Multiorganversagen.

Messung der differentiellen Genexpression als Basis für den Traingsset:
Für die Messung der differentiellen Genexpression zur Unterscheidung zwischen nichtinfektiösen und infektiösen Ursachen eines Multiorganversagens wurden Untersuchungen von Vollblutproben von insgesamt 57 Patienten, welche auf operativen Intensivstationen behandelt wurden, durchgeführt. Die gesamten Genexpressionsdaten bildeten die Grundlage füt die Erstellung des Trainsgsets der Genaktiviäts-Klassifikatoren.

Es wurden Vollblutproben von 31 Patienten abgenommen, welchen im Rahmen ihrer intensivmedizinischen Betreuung ein infektiöses MOV [klassifiziert nach 8] entwickelten.

Weiterhin wurden Vollblutproben von 26 Patienten abgenommen, welche im Rahmen ihrer intensivmedizinischen Betreuung ein nichtinfektiöses MOV [klassifiziert nach 8] entwickelten.

Außerdem wurden Vollblutproben von 18 Patienten abgenommen, welche im Laufe ihrer intensivmedizinischen behandelt wurden (im folgenden ITS - Kontrollen).

Als Referenzproben dienten die totale RNA aus Zelllinien SIG-M5.

Ausgewählte Charakteristika der drei Patientengruppen sind in der Tabelle 2 dargestellt. Dabei werden Angaben zum Alter, Geschlecht, und dem SOFA-Score als Maß für die Funktion der Organsysteme gemacht. Gleichfalls sind die Plasmaproteinspiegel von Procalcitonin (PCT) und CRP sowie die Zahl der Leukozyten der Patienten angegeben.

Alle Patientenproben wurden mit der Referenzprobe jeweils auf einem Microarray ko-hybridisiert.

**Tabelle 2: Daten der Patientengruppen**

| | | **ITS Kontrollen** | **Nichtinfektiöses MOV** | **Infektiöses MOV** |
|---|---|---|---|---|
| **Anzahl der Patienten** | | 18 | 26 | 31 |
| **Geschlecht M/W** | | 16/2 | 15/11 | 17/14 |
| **Alter** [Jahre] | | 65 (15) | 69 (10) | 60 (17) |
| **APACHE-II Score** [Punkte] | | 10* (2.8) | 14.9 (3.4) | 14 (10) |
| **SOFA Score** [Punkte] | | 3.4* (1.7) | 8*(3) | 10*(3) |
| **Zahl der Organdysfunktionen** | | - | 3 (1) | 3 (1) |
| **PCT** [ng/ml] | | 0.56* (0.8) | 3.8 (6.7) | 3.1 (7.7) |
| **CRP** [µg/I] | | 68.5* (27.5) | 80.2* (90.2) | 188* (168) |
| **WBC** [no/I] | | 8,088* (3,554.2) | 12,300 (6,925) | 13,200 (8,150) |

| | | | | |
|---|---|---|---|---|
| *p<0.05 | | | | |

### Experimentelle Beschreibung:

Nach Abnahme des Vollblutes wurde die totale RNA der Proben unter Anwendung des PAXGene Blood RNA Kit gemäß den Vorgaben des Herstellers (Qiagen) isoliert.

### Zellkultivierung

Für die Zellkultivierung (Kontrollproben) wurden 19 Kryozellkulturen (SIGM5) (eingefroren in flüssigem Stickstoff) genutzt. Die Zellen wurden jeweils mit 2 ml Iscove's Medium (Biochrom AG) beimpft ergänzt mit 20% fetalen Kälber Serum (FCS). Die Zellkulturen wurden anschliessend für 24 Stunden bei 37° C unter 5% CO2 in 12-well Platten inkubiert. Danach wurde der Inhalt von 18 Wells in 2 Teile mit jeweils dem gleichen Volumen geteilt, sodass schliesslich 3 Platten des gleichen Formats (insgesamt 36 Wells) zur Verfügung standen. Die Kultivierung wurde anschliessend für 24 Stunden unter den gleichen Bedingungen fortgeführt. Im Anschluss daran wurden die resultierenden Kulturen von 11 Wells jeder Platte vereint und zentrifugiert (1000 x g, 5 min, Raumtemperatur). Der Überstand wurde verworfen und das Zellpellet in 40 ml des o.g. Mediums gelöst. Diese 40 ml gelöste Zellen wurden in zwei 250 ml Kolben zu gleichen Teilen aufgeteilt und nach 48 Stunden Inkubation und Zugabe von 5 ml des o.g. Mediums wiederum inkubiert. Von den restlichen 2 ml der zwei verbleibendenden Platten wurden 80 µl in leere Wells der gleichen Platten gegeben, welche bereits vorher mit 1 ml des o.g. Mediums präpariert waren. Nach 48 Stunden Inkubation wurde nur eine der 12 Weil-Platten wie folgt prozessiert: Aus jedem Weil wurden 500 µl entnommen und vereint. Die daraus resultierenden 6 ml wurden in einen 250 ml Kolben gegeben, welcher ca. 10 ml frisches Medium enthielt. Dieses Gemisch wurde mit 1000 x g 5 Minuten bei Raumtemperatur zentrifugiert und in 10 ml des o.g. Mediums gelöst. Die anschliessende Zellzählung ergab folgendes Ergebnis: 1,5 x 107 Zellen pro ml, 10 ml Gesamtvolumen, Gesamtzahl der Zellen: 1,5 x 108. Da die Zellzahl noch nicht ausreichend war, wurden 2,5 ml des o.g. Zellsuspension in 30 ml des o.g. Mediums in einen 250 ml (75 cm²) Kolben gegeben (insgesamt 4 Kolben). Nach 72 Sunden Inkubationszeit wurden jeweils 20 ml frischen Mediums in die Kolben gegeben. Nach folgender 24-stündiger Inkubation erfolgte die Zellzählung wie oben beschrieben, die eine Gesamtzellzahl von 3,8 x 10⁸ Zellen ergab. Um die gewünschte Zellzahl von 2 x 106 Zellen zu errreichen wurden die Zellen in 47,5 ml des o.g. Mediums in 4 Kolben resuspendiert. Nach einer Inkubationszeit von 24 Stunden wurden die Zellen zentrifugiert und zweimal mit Phosphatpuffer ohne Ca²⁺ und Mg²⁺ (Biochrom AG) gewaschen.

Die Isolation der totalen RNA erfolgt mittels des NucleoSpin RNA L Kits (Machery&Nagel) entsprechend den Angaben des Herstellers. Die oben beschriebene Prozedur wurde wiederholt bis die erforderliche Zellzahl erreicht wurde. Dies war erforderlich, um die erforderliche Menge von 6 mg totale RNA zu erreichen, was etwa einer Effizienz von 600 µg RNA pro 108 Zellen entspricht.

Reverse Transkription / Markierung / Hybridisierung Nach Abnahme des Vollblutes wurde die totale RNA der Proben unter Verwendung des PAXGene Blood RNA Kits (PreAnalytiX) gemäss den Vorgaben des Herstellers isoliert und auf ihre Qualität geprüft. Von jeder Probe wurden 10 µg totale RNA aliquotiert und zusammen mit 10 µg total RNA aus SIGM5-Zellen als Referenz-RNA zu komplementärer DNA (cDNA) mit der reversen Transkriptase Superscript II (Invitrogen) umgeschrieben und die RNA anschließend durch alkalische Hydrolyse aus dem Ansatz entfernt. Im Reaktionsansatz wurde ein Teil des dTTP durch AminoallyldUTP (AA-dUTP) ersetzt, um später die Kopplung des Fluoreszenzfarbstoffes an die cDNA zu ermöglichen.

Nach der Aufreinigung des Reaktionsansatzes wurden die cDNA der Proben und Kontrollen mit den Fluoreszenzfarbstoffen Alexa 647 und Alexa 555 kovalent markiert und auf einem Microarray der Firma SIRS-Lab hybridisiert. Auf dem verwendeten Microarray befinden sich 5308 immobilisierte Polynukleotide mit einer Länge von 55 - 70 Basenpaaren, die jeweils ein humanes Gen repräsentieren und Kontrollspots zur Qualitätssicherung. Ein Microarray unterteilt sich in 28 Subarrays mit einem Raster von 15x15 Spots.

Die Hybridisierung und das anschliessende Waschen bzw. Trocknen wurde in der Hybridisierungsstation HS 400 (Tecan) nach Angaben des Herstellers über 10,5 Stunden bei 42°C durchgeführt. Die verwendete Hybridisierungslösung besteht aus den jeweiligen gelabelten cDNA-Proben, 3,5x SSC (1x SSC enthält 150 mM Natriumchlorid und 15 mM Natriumcitrat), 0,3% Natriumdodecylsulfat (V/V) 25% Formamid (V/V) und je 0,8 µg µl-1 cot-1 DNA, Hefe t-RNA und poly-A RNA. Das anschliessende Waschen der Mikroarrays wurde mit nachfolgendem Programm bei Raumtemperatur in durchgeführt: je 90 Sekunden spülen mit Waschpuffer 1 (2x SSC, 0,03% Natriumdodecylsulfat), mit Waschpuffer 2 (1x SSC) und abschließend mit Waschpuffer 3 (0,2x SSC). Danach wurden die Mikroarrays unter einem Stickstoffstrom mit einem Druck von 2,5 bar bei 30°C über 150 Sekunden getrocknet.

Nach der Hybridisierung wurden die Hybridisierungssignale der Microarrays mit einem GenePix 4000B Scanner (Axon) ausgelesen und die Expressionsverhältnisse der differenziert exprimierten Gene mit der Software GenePix Pro 4.0 (Axon) bestimmt.

### Auswertung:

Für die Auswertung wurde die mittlere Intensität eines Spots als der Medianwert der zugehörigen Spotpixel bestimmt.

### Korrektur systematischer Fehler:

Die Korrektur systematischer Fehler erfolgte nach dem Ansatz von Huber et al. [34]. Dabei wurden der additive und der multiplikative Bias innerhalb eines Microarrays aus 70% der vorhandenen Genproben geschätzt. Für alle weiteren Berechnungen wurden die Signale mittels arcus sinus hyperbolicus transformiert.

Für die Analyse wurden die normalisierten und transformierten relativen Verhältnisse der Signale der Patientenproben gegen die allgemeine Kontrolle berechnet. D.h. für das j-te Gen des n-ten Patienten ergab die Berechnung den Wert Gj,n = arcsinh(Scy5(j,n)) - arcsinh(Scy3(j,n)), wobei [SCy3(j,n), SCy5(j,n)] das zugehörige Signalpaar bezeichnet. War für einen Patienten ein Spot nicht auswertbar (z.B. Fleck auf dem gescannten Bild), so wurde der zugehörige Wert als nicht vorhanden (,missing value') gekennzeichnet.

### Statistischer Vergleich:

Für den Vergleich wurde der zweiseitige Zweistichproben Student-Test pro Gen verwendet. Beide Stichproben enthielten die Werte der Patientengruppen nichtinfektiöses MOV bzw. infektiöses MOV. Für die Auswahl der differenziert exprimierten Gene wurde der zugehörige p-Wert bewertet. Für die Gruppe der ausgewählten Gene war der zugehörige p-Wert kleiner als 0.05.

**Tabelle 3: Signifikant gesteigerte Genaktivitäten in Proben von Patienten mit infektiösem MOV, im Vergleich zu den Genaktivitäten von Patienten mit nichtinfektiösem MOV**

| **GenBank Accession-Number** | **p-Wert** | **Mittlerer normalisierter und transformierter Expressionswert** | | **Standardabwelchung** | | **Sequenz-ID** |
|---|---|---|---|---|---|---|
| | | **Gruppe der Patienten mit nichtinfektlösem /MOV** | **Gruppe der Patienten mit Infektlösem MOV** | **Gruppe der Patienten mit nichtinfektlösem /MOV** | **Gruppe der Patienten mit Infektlösem MOV** | |
| N32857 | 0,00 | -2,99 | 0,20 | 1,42 | 2,78 | 1 |
| N32853 | 0,00 | -0,85 | 1,60 | 2,15 | 2,89 | 2 |
| N32495 | 0,00 | -2,38 | -0,56 | 1,37 | 0,40 | 3 |
| AI701077 | 0,01 | -0,33 | 1,46 | 0,17 | 3,08 | 4 |
| M87790 | 0,00 | 1,18 | 2,93 | 1,13 | 1,24 | 5 |
| AI559317 | 0,01 | 0,20 | 1,83 | 0,54 | 2,60 | 6 |
| N34897 | 0,00 | -2.60 | -1,05 | 1,61 | 0,54 | 7 |
| AA907084 | 0,02 | 0,53 | 1,94 | 0,49 | 2,58 | 8 |
| N45223 | 0,00 | -2,88 | -1,54 | 1,24 | 0,57 | 9 |

Die in Tabelle 3 aufgeführten GenBank Accession Nummern (Internet-Zugang über http://www.ncbi.nlm.nih.gov/) der einzelnen Sequenzen sind in dem dieser Anmeldung angefügten Sequenzprotokol im Einzelnen jeweils einer Sequenz ID (SEQ ID 1 bis zur SEQ ID 9) zugeordnet.

Ferner sind die Sequenzen darin im Einzelnen offenbart.

Dieses Sequenzprotokoll ist Bestandteil der Beschreibung der vorliegenden Erfindung.

Zusätzlich sind diese Sequenzen in der deutschen Patentanmeldung Nr. DE 102004049897.0 offenbart.

### Erstellung der Genaktiviäts-Klassifikatoren (Trainingsset)

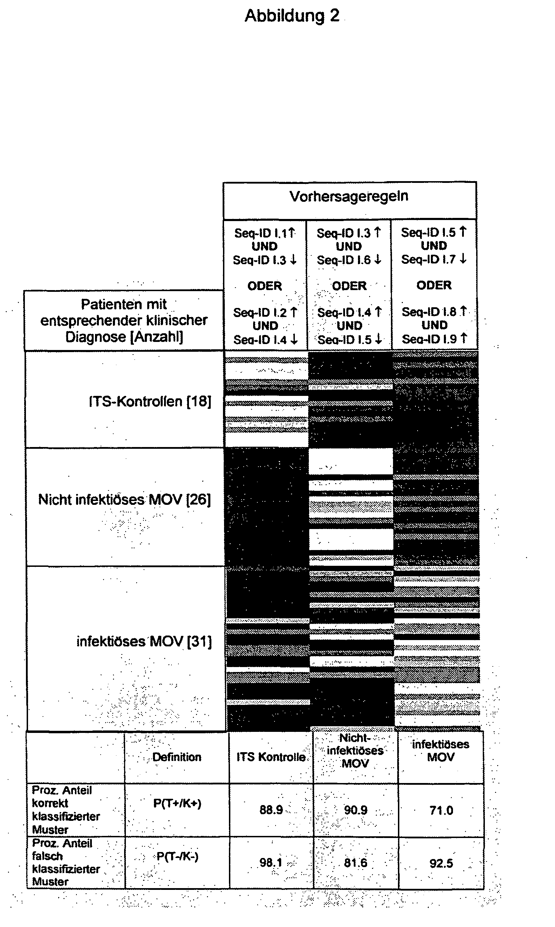

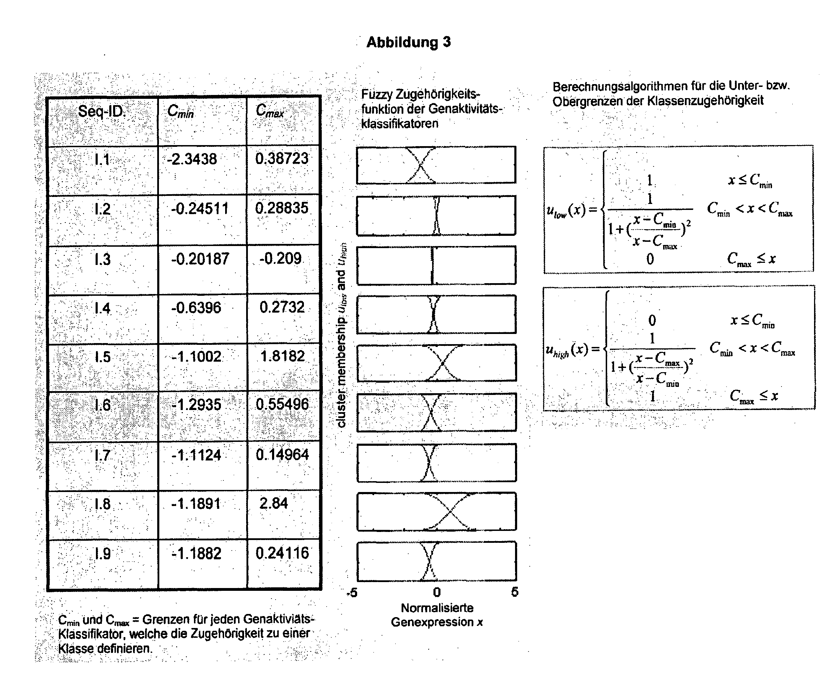

Ausgehend von allen gemessenen Genaktiviäten wurden mittels der Software MediPred (Fa. Biocontrol Jena GmbH) Genaktiviäts-Klassifikatoren und Auswahl-Regeln erstellt, die eine Klassifizierung der Genexpressionsmuster in die Klassen infektiöses MOV und nichtinfektiöses MOV ermöglichen (Abbildung 2). Dabei wurden Gen für Gen Schwellenwerte für hohe und niedrige Expression bestimmt (in Abbildung 3 mit Cₘᵢₙ und Cₘₐₓ bezeichnet) und Gene mit typischem und robustem Verhalten der Genexpression pro Patientenklasse extrahiert.

### Testung der Genaktiviätsklassifikatoren an noch nicht klassifzierten Genexpressionsprofilen (Testset)

Die festgelegten Genaktivitäs-Klassifikatoren wurden anhand der definierten Auswahlregeln an einem Testset von insgesamt 190 noch nicht klassifizierten Genexpressionsmustern von ITS-Kontrollen (56), Patienten mit nichtinfektiösem MOV (75) bzw. Patienten mit infektiösem MOV (59) getestet und anhand klinischer Daten validiert.

Hierfür wurden Genexpressionsprofile von Patienten ausgewählt, welche klinisch als ITS-Kontrolle oder nichtinfektiöses MOV oder infektiöses MOV diagnostiziert wurden. Konnte in dem anschließenden Vergleich mit dem Trainingsset festgestellt werden, dass die zu klassifizierenden Expressionsmuster innerhalb der mittels der Auswahlregeln festgelegten Expressionsgrenzen der entsprechenden Genaktiviäts-Klassifikatoren (bei gleichzeitigem Zutreffen der Auswahlregeln gelegen haben), dann wurde das zu klassifizierende Expressionsmuster der entsprechenden Klasse zugeordnet.

Aus Tabelle 4 ist die Zugehörigkeit des Testsets zu den entsprechenden Klassen ersichtlich. Demzufolge konnten 86% der ITS-Kontrollen, 80% der Patienten mit nichtinfektiösem Multiorganversagen sowie 63% der Patieten mit infektiösem Multiorganversagen korrekt in die entsprechende Klasse eingeordnet werden.

**Tabelle 4: Prozentualer Anteil von 190 zu klassifizierende Genexpressionsprofile in die Klassen IST-Kontrolle, nichtinfektiöses MOV und infektiöses MOV**

| Patientengruppen | | ITS- Kontrollen | Nichtinfektiöses Multiorganversagen | Infektiöses Multiorganversagen |
|---|---|---|---|---|
| Zahl der zu klassifizierenden Genexpressionsprofile | | 56 | 75 (mehere Tage von 16 Patienten) | 59 (mehrere Tage von 35 Patienten) |
| Zugehörigkeit zu den | ITS-Kontrollen Nichtinfektiöses Multiorganversagen | 86 | 4 | 14 |
| | | 13 | 80 | 16 |
| Klassen [%] | Infektiöses Multiorganversagen | 2 | 16 | 63 |

Damit sind die Genaktiviätsklassifikatoren in Verbindung mit den Auswahlregeln für die Erfindung anwendbar.

### Referenzen

1. Natanson C (1997) Anti-inflammatory therapies to treat sepsis and septic shock: A reassessment. Crit Care Med 25: 1095-1099
2. Geiger K (1995) Frühparameter für Multiorgandysfunktionssyndrom. in Hartenauer U (ed.) Sepsis in der Frühphase München MMV Medizin Verlag 19-25
3. Knaus WA , Draper EA, Wagner DP, Zimmermann JE (1985) Prognosis in acute organ-system failure. Ann Surg 202: 658-693
4. Goris RI, Bockhorst TP, Nuytinck JKS (1995) Mulitiple organ failure. Arch Surg 120:1109-1115
5. Vincent JL, Moreno R, Takala J, et al. (1996) The SOFA (Sepsis-related Organ Failure Assessment) score to describe organ dysfunction/failure. On behalf of the Working Group on Sepsis-Related Problems of the European Society of Intensive Care Medicine, Intensive Care Med. Jul 22(7):707-10.
6. Pfeiffer L, Ehrhardt N, Kretschmar R, et al. (1996) Endotoxinämie und Multiorganversagen nach Polytrauma. Anaesthesiol Reanimat 21: 91-96
7. Schlag G, Redl H (1993) Organ in shock, early organ failure, late organ failure., in Schlag G and Redl H (eds.) Pathophysiology of shock, sepsis, and organ failure Berlin Heidelberg Springer-Verlag, 1-4
8. Bone RC, Balk RA, Cerra FB, et al. (1992) The ACCP/SCCM Consensus Conference Committee (1992) Definitions for Sepsis and organ failure and guidelines for the use of innovative therapies in Sepsis. Chest 101:1656-1662; und Crit Care Med 1992; 20: 864-874.
9. Levy MM, Fink M, Marshall JC, et al. (2003) For the International Sepsis Definitions Conference: 2001 SCCM/ESICM/ACCP/ATS/SIS International Sepsis Definitions Conference. Crit Care Med. Apr;31 (4):1250-6
10. http://chirinn.klinikum.uni-muenchen.de/forschung/for_01_14_04.html, Stand Otober 2004, modifiziert
11.Marik PE. (1993) Gastric intramucosal pH. A better predictor of multiorgan dysfuction syndrom and death than oxygen derived variables in patients with sepsis. CHEST 104:, 225-229
12. Bemardin G , Pradier C, Tiger F, et al. (1996) Blood pressure and arterial lactate level are early indicators of short-term survival in human septic shock. Intensiv Care Med 22: 17-25;
13.Marecaux G, Pinsky MR, Dupont E, et al. (1996) Blood lactate levels are better prognostic indicators than TNF and IL-6 levels in patients with septic shock. Intensiv Care Med 22: 404-408
14.Duswald KH , Jochum M , Schramm W , Fritz H (1985) Released granulocytic elastase: an indicator of pathobiochemical alterations in septicemia after abdominal surgery. Surgery 98: 892-899
15. Nuytinck JKS, Goris RI, Redl H, et al. (1986) Posttraumatic complications and inflammatory mediators. Arch Surg 121: 886-890
16.Nast-Kolb D, Jochum M, Waydlas C, et al. (1991) Die Wertigkeit biochemischer Faktoren beim Polytrauma. Hefte Unfallheilkunde 215: 215
17. Hack CE, de Groot ER , Felt-Bersma RJ , et al. (1989): Increased plasma levels of interleukin-6 in sepsis" Blood 74: 1704-1710
18. Patel RT, Deen KI, Youngs D, et al. (1994) Interleukin 6 is a prognostic indicator of outcome in severe intra-abdominal sepsis. Br J Surg 81:1306-1308
19.Southern EM (1974) An improved method for transferring nucleotides from electrophoresis strips to thin layers of ion-exchange cellulose. Anal Biochem 62:317-318
20. Gillespie D, Spiegelman S (1965) A quantitative assay for DNA-RNA hybrids with DNA immobilized on a membrane. J Mol Biol 12:829-842
21. Lennon GG, Lehrach H (1991) Hybridization analyses of arrayed cDNA libraries. Trends Genet 7: 314-317
22.Kafatos FC, Jones CW, Efstratiadis A (1979) Determination of nucleic acid sequence homologies and relative concentrations by a dot hybridization procedure. Nucl Acid Res 7:1541-1552
23. Fodor SP, Read JL, Pirrung MC, Stryer L, Lu AT, Solas D (1991) Lightdirected, spatially addressable parallel chemical synthesis. Science 251:767-773
24.Pease AC, Solas D, Sullivan EJ, Cronin MT, Holmes CP, Fodor SP (1994) Light-generated oligonucleotide arrays for rapid DNA sequence analysis. Proc Natl Acad Sci USA 91:5022-5026
25.Schena M, Shalon D, Davis RW, Brown PO (1995) Quantitative monitoring of gene expression pattems with a complementary DNA microarray. Science 270:467-470
26. Golub TR, Slonim DK, Tamayo P, et al. (1999) Molecular classification of cancer: class discovery and class prediction by gene expression monitoring. Science 286:531-537
27.Alizadeh AA, Eisen MB, Davis RE, et al. (2000) Distinct types of diffuse large B-cell lymphoma identified by gene expression profiling. Nature 403:503-51
28.Feezor RJ, Baker HV, Xiao W, et al. (2004) Genomic and Proteomic Determinants of outcome in patients undergoing thoracoabdominal aortic aneurysm repair. Journal of Immunology 172 (11): 7103-7109
29. Rademacher md; mCsHANE Im; Simon R (2002) A paradigm for class prediction using gene expression profiles. J.Comput.Biol. 9:505-511
30. Li L, Darden TA, Weinberg CR, Levine AJ et al. (2001) Gene assesment and sample classification for gene expression data using a genetic algorith/k-nearest neighbor method. Comb. Chem. High Throuput Screen. 4:727 7-39
31.Zhang H, Yu C-Y, Singer B etal. (2001) Recursive partitioning for tumor classification with gene expression microarray data. Proc. Nat. Acad. Sci. USA 2001, 98:6730-6735
32.Furey TS, Cristianni N, Duffy N (2000) Support vector machine classification and validation of cancer tissue samples using microarray expression data. Bioinf. 16:906-914
33. Simon RM, Kom EL, McShane L et al. (2004) Design and analysis of DNA microarary investigations. Springer-Verlag, New York, ISBN 0-387-00135-2
34. Huber W, Heydebreck A, Sueltmann H, et al. (2003) Parameter estimation for the calibration and variance stabilization of microarray data. Stat. Appl. in Gen. and Mol. Biol., Vol. 2, Issue 1, Article 3

## Patentansprüche

1. Verwendung von Polynukleotiden, welche ausgewählt sind aus der Gruppe bestehend aus: SEQ ID I.1, SEQ ID I.2, SEQ ID I.3, SEQ ID I.4, SEQ ID I.5, SEQ ID I.6, SEQ ID I.7, SEQ ID I.8 und SEQ ID I.9 oder Teilsequenzen davon mit einer Länge von 20 bis 200 Nukleotiden als Genaktivitätsmarker zur Klassifizierung von Patienten als "nicht infiziert ohne Multiorganversagen" oder als "nicht an infektiösem Multiorganversagen erkrankt" oder als an "infektiösem Multiorganversagen erkrankt".

2. Verwendung nach Anspruch 1, wobei die Teilsequenzen der Polynukleotide eine Länge von 20-80 Nukleotiden haben.

3. Verwendung nach Anspruch 1-2, **dadurch gekennzeichnet, dass** eine Überexpression von SEQ ID I.1 und eine Unterexpression von SEQ ID 1.3 oder eine Überexpression von SEQ ID I.2 und eine Unterexpression von SEQ ID I.4 die Klassifikation "keine Infektion und ohne Multiorganversagen" anzeigt;
eine Überexpression von SEQ ID I.3 und eine Unterexpression von SEQ ID I.6 oder eine Überexpression von SEQ ID I.4 und eine Unterexpression von SEQ ID I.5 die Klassifikation "nichtinfektiöses Multiorganversagen" anzeigt;
eine Überexpression von SEQ ID I.5 und eine Unterexpression von SEQ ID I.7 oder eine Überexpression von SEQ ID I.8 und eine Unterexpression von SEQ ID 1.9 die Klassifikation "infektiöses Multiorganversagen" anzeigt.

4. Verwendung nach Anspruch 1-3, **dadurch gekennzeichnet, daß** die Polynukleotidsequenzen oder -teilsequenzen zumindest teilweise durch synthetische Analoga, Aptamere und/oder Peptidonukleinsäuren ersetzt werden.

5. Verwendung nach Anspruch 1-4 in einem Microarray.

6. Verfahren zur Klassifizierung von Patienten, die an infektiösem bzw. nicht infektiösem Multiorganversagen erkrankt sind, umfassend die Schritte:
Isolation der mRNA aus einer Patientenprobe;
Markieren der mRNA mit einer detektierbaren Einheit;
In-Kontakt-Bringen der markierten mRNA mit den Genaktivitätsmarkem gemäß Anspruch 1, so dass die einzelnen Genaktivitätsmarker auch nach dem In-Kontakt-Bringen getrennt voneinander vorliegen;
Waschen der hybridisierten Genaktivitätsmarker,
Anregen der detektierbaren Einheit, so dass diese ein Signal emittiert;
Auslesen der Hybridisierungssignale für jeden einzelnen Genaktivitätsmarker und Vergleich mit einer Referenzprobe eines gesunden Patienten, wobei
eine Überexpression von SEQ ID I.1 und eine Unterexpression von SEQ ID I.3 oder eine Überexpression von SEQ ID I.2 und eine Unterexpression von SEQ ID I.4 die Klassifikation "keine Infektion und ohne Multiorganversagen" anzeigt;
eine Überexpression von SEQ ID I.3 und eine Unterexpression von SEQ ID I.6 oder eine Überexpression von SEQ ID I.4 und eine Unterexpression von SEQ ID I.5 die Klassifikation "nichtinfektiöses Organversagen" anzeigt; und
eine Überexpression von SEQ ID I.5 und eine Unterexpression von SEQ ID I.7 oder eine Überexpression von SEQ ID I.8 und eine Unterexpression von SEQ ID I.9 die Klassifikation "infektiöses Multiorganversagen" anzeigt.

7. Verfahren nach Anspruch 6, **dadurch gekennzeichnet, dass** die Patientenprobe Körperflüssigkeiten, insbesondere Blut, Liquor, Urin, Ascitesflüssigkeit, Seminalflüssigkeit, Speichel, Punktat, Zellinhalt oder eine Mischung daraus umfasst.

8. Verfahren nach Anspruch 6 oder 7, wobei das Verfahren mit einem Microarray durchgeführt wird.

9. Microarray zur Klassifizierung von Patienten mit infektiösem oder nicht infektiösem Multiorganversagen, bestehend aus Polynukleotide mit den Sequenzen SEQ ID I.1, SEQ ID I.2, SEQ ID I.3, SEQ ID I.4, SEQ ID I.5, SEQ ID I.6, SEQ ID I.7, SEQ ID I.8 und SEQ ID I.9 oder Teilsequenzen davon mit einer Länge von 20 bis 200 Nukleotiden.

10. Vorrichtung zur Klassifizierung von Patienten mit infektiösem oder nicht infektiösem Multiorganversagen, umfassend:
ein Modul für die Probenvorbereitung der *in vitro* gewonnenen Patientenprobe, ein Modul für die Hybridisierung der Patientenprobe mit Genaktiviätssonden, die von den Genaktivitäts-Klassifikatoren abgeleitet sind, bestehend aus Genaktivitäts-Klassifikatoren mit den sequenzen SEQ ID I.1, SEQ ID I.2, SEQ ID 1.3, SEQ ID 1.4, SEQ ID 1.5, SEQ ID I.6, SEQ ID I.7, SEQ ID I.8 und SEQ ID I.9 oder Teilsequenzen davon mit einer Länge von 20 bis 200 Nukleotiden.
ein Modul für die Auslesung der Hybridisierungssignale,
ein Modul für die Bildanalyse der ausgelesenen Hybridisierungssignale,
ein Modul, das den automatischen Vergleich mit gespeicherten Genaktiviätsklassifikatoren von gesunden Patienten ermöglicht und
ein Modul, dass die Anzeige des resultierenden Vergleichs ermöglicht.

## Claims

1. Use of polynucleotides selected from the group consisting of: SEQ ID 1.1, SEQ ID I.2, SEQ ID I.3, SEQ ID I.4, SEQ ID 1.5, SEQ ID I.6, SEQ ID 1.7, SEQ ID 1.8, and SEQ ID I.9 or partial sequences thereof having a length of 20 to 200 nucleotides as gene activity markers for the classification of patients into "not infected without multiple organ failure", "not suffering from infectious multiple organ failure", or "suffering from infectious multiple organ failure."

2. The use according to claim 1, wherein the length of the partial sequences is 20 - 80 nucleotides.

3. The use according to claim 1 - 2, **characterized in that** an overexpression of SEQ ID I.1 and an underexpression of SEQ ID I.3 or an overexpression of SEQ ID I.2 and an underexpression of SEQ ID I.4 indicates the classification "no infection and without multiple organ failure";
an overexpression of SEQ ID I.3 and an underexpression of SEQ ID I.6 or an overexpression of SEQ ID I.4 and an underexpression of SEQ ID I.5 indicates the classification "non-infectious multiple organ failure";
an overexpression of SEQ ID I.5 and an underexpression of SEQ ID I.7 or an overexpression of SEQ ID I.8 and an underexpression of SEQ ID I.9 indicates the classification "infectious multiple organ failure."

4. The use according to claim 1 - 3, **characterized in that** the polynucleotide sequences or the partial polynucleotide sequences are replaced, at least partially, by synthetic analogues, aptamers and/or peptidonucleic acids.

5. The use according to claim 1 - 4 in a microarray.

6. A method for classifying patients suffering from infectious and non-infectious multiple organ failure, respectively, comprising the following steps:
isolation of mRNA from a patient sample;
labeling the mRNA with a detectable unit;
contacting the labeled mRNA with the gene activity markers according to claim 1, so that the individual gene activity markers are separated from each other even after the contacting step;
washing the hybridized gene activity markers;
stimulating the detectable unit so that the latter emits a signal;
reading out the hybridization signals for each of the individual gene activity markers and comparing them with a reference sample of a healthy patient, wherein
an overexpression of SEQ ID I.1 and an underexpression of SEQ ID I.3 or an overexpression of SEQ ID I.2 and an underexpression of SEQ ID I.4 indicates the classification "no infection and without multiple organ failure";
an overexpression of SEQ ID I.3 and an underexpression of SEQ ID I.6 or an overexpression of SEQ ID I.4 and an underexpression of SEQ ID I.5 indicates the classification "non-infectious organ failure"; and
an overexpression of SEQ ID I.5 and an underexpression of SEQ ID I.7 or an overexpression of SEQ ID I.8 and an underexpression of SEQ ID I.9 indicates the classification "infectious multiple organ failure."

7. The method according to claim 6, **characterized in that** the patient sample comprises body fluids, in particular blood, liquor, urine, ascitic fluid, seminal fluid, saliva, puncture fluid, cell content, or a mixture thereof.

8. The method according to claim 6 or 7, wherein the method is carried out with the aid of a microarray.

9. A microarray for classifying patients with infectious and non-infectious multiple organ failure, respectively, consisting of polynucleotides having the sequences SEQ ID I.1, SEQ ID I.2, SEQ ID I.3, SEQ ID I.4, SEQ ID I.5, SEQ ID I.6, SEQ ID I.7, SEQ ID 1.8, and SEQ ID I.9 or partial sequences thereof having a length of 20 to 200 nucleotides.

10. A device for classifying patients with infectious or non-infectious multiple organ failure, comprising:
a module for the sample preparation of the patient sample obtained *in vitro,* a module for hybridizing the patient sample with gene activity probes derived from the
gene activity classifiers, consisting of gene activity classifiers having the sequences SEQ ID I.1, SEQ ID I.2, SEQ ID I.3, SEQ ID I.4, SEQ ID I.5, SEQ ID I.6, SEQ ID I.7, SEQ ID I.8, and SEQ ID I.9 or partial sequences thereof having a length of 20 to 200 nucleotides,
a module for reading out the hybridization signals,
a module for the image analysis of the read hybridization signals,
a module allowing for automatic comparison with stored gene activity classifiers of healthy patients, and
a module for displaying the resulting comparison.

## Revendications

1. Utilisation de polynucléotides, lesquels sont sélectionnés parmi le groupe consistant en : SEQ ID I.1, SEQ ID I.2, SEQ ID I.3, SEQ ID I.4, SEQ ID 1.5, SEQ ID I.6, SEQ ID 1.7, SEQ ID I.8 et SEQ ID I.9 ou des séquences partielles de ces dernières, avec une longueur de 20 à 200 nucléotides, comme marqueur d'activité génique pour la classification de patients comme « non infecté, sans défaillance organique multiple », « non atteint de défaillance organique multiple infectieuse », ou comme « atteint d'une défaillance organique multiple infectieuse ».

2. Utilisation selon la revendication 1, dans laquelle les séquences partielles de polynucléotides ont une longueur de 20 à 80 nucléotides.

3. Utilisation selon les revendications 1 ou 2, **caractérisée en ce qu'**une surexpression de la SEQ ID I.1 et une sous-expression de la SEQ ID 1.3 ou une surexpression de la SEQ ID I.2 et une sous-expression de la SEQ ID I.4 indiquent la classification « pas d'infection et pas de défaillance organique multiple » ;
**en ce qu'**une surexpression de la SEQ ID I.3 et une sous-expression de la SEQ ID I.6 ou une surexpression de la SEQ ID 1.4 et une sous-expression de la SEQ ID I.5 indiquent la classification « défaillance organique multiple non infectieuse » ;
**en ce qu'**une surexpression de la SEQ ID I.5 et une sous-expression de la SEQ ID I.7 ou une surexpression de la SEQ ID I.8 et une sous-expression de la SEQ ID I.9 indiquent la classification « défaillance organique multiple infectieuse ».

4. Utilisation selon les revendications 1 à 3, **caractérisée en ce que** les séquences ou les séquences partielles de polynucléotides sont remplacées au moins en partie par des analogues synthétiques, des aptamères et/ou des acides peptidonucléiques.

5. Utilisation selon les revendications 1 à 4 dans une puce de réactifs biochimiques.

6. Procédé de classification de patients atteints d'une défaillance organique multiple infectieuse ou non infectieuse, comprenant les étapes :
isolation de l'ARN-m d'un échantillon d'un patient ;
marquage de l'ARN-m avec une unité détectable ;
mise en contact de l'ARN-m marqué avec les marqueurs d'activité génique selon la revendication 1, de telle sorte que les différents marqueurs d'activité génique se trouvent séparés les uns des autres, même après la mise en contact ;
lavage des marqueurs d'activité génique hybridés ;
excitation de l'unité détectable, de telle sorte que celle-ci émette un signal ;
lecture des signaux d'hybridation pour chacun des marqueurs d'activité génique et comparaison avec un échantillon de référence d'un patient sain, sachant que
une surexpression de la SEQ ID I.1 et une sous-expression de la SEQ ID I.3 ou une surexpression de la SEQ ID 1.2 et une sous-expression de la SEQ ID 1.4 indiquent la classification « pas d'infection et sans défaillance organique multiple » ;
une surexpression de la SEQ ID 1.3 et une sous-expression de la SEQ ID I.6 ou une surexpression de la SEQ ID I.4 et une sous-expression de la SEQ ID I.5 indiquent la classification « défaillance organique multiple non infectieuse » ; ; et
une surexpression de la SEQ ID I.5 et une sous-expression de la SEQ ID I.7 ou une surexpression de la SEQ ID I.8 et une sous-expression de la SEQ ID I.9 indiquent la classification « défaillance organique multiple infectieuse ».

7. Procédé selon la revendication 6, **caractérisé en ce que** l'échantillon du patient comprend des liquides corporels, en particulier du sang, du liquide céphalorachidien, de l'urine, du liquide d'ascite, du liquide séminal, de la salive, du liquide obtenu par ponction, du contenu de cellules ou un mélange de ceux-ci.

8. Procédé selon la revendication 6 ou 7, le procédé étant exécuté avec une puce à réactifs biochimiques.

9. Puce à réactifs biochimiques pour la classification de patients avec des défaillances d'organes multiples infectieuses ou non infectieuses, consistant en des polynucléotides avec les séquences SEQ ID I.1, SEQ ID 1.2, SEQ ID I.3, SEQ ID I.4, SEQ ID I.5, SEQ ID I.6, SEQ ID 1.7, SEQ ID 1.8 et SEQ ID I.9 ou des séquences partielles de celles-ci avec une longueur de 20 à 200 nucléotides.

10. Dispositif pour la classification de patients avec des défaillances d'organes multiples infectieuses ou non infectieuses, comprenant :
un module pour la préparation de l'échantillon de patient obtenu *in vitro,* un module pour l'hybridation de l'échantillon du patient avec des sondes d'activité génique qui dérivent des classificateurs d'activité génique, consistant en des classificateurs d'activité génique avec les séquences SEQ ID I.1, SEQ ID I.2, SEQ ID I.3, SEQ ID I.4, SEQ ID I.5, SEQ ID I.6, SEQ ID I.7, SEQ ID I.8 et SEQ ID I.9 ou des séquences partielles de celles-ci avec une longueur de 20 à 200 nucléotides,
un module pour la lecture des signaux d'hybridation,
un module pour l'analyse d'image des signaux d'hybridation lus,
un module qui permet la comparaison automatique avec des classificateurs d'activité génique enregistrés de patients sains,
un module qui permet l'affichage de la comparaison résultante.
